# EUROPEAN PATENT APPLICATION

(11) **EP 4 407 319 A1**
(43) Date of publication of application: **31.07.2024**
(21) Application number: 23153713.5
(22) Date of filing: 27.01.2023
(51) Int. Cl.: G01N 33/86

(54) **SYSTEM FOR DETERMINING A FUNCTIONAL FIBRINOGEN CONCENTRATION**

(71) Applicant: Hemeo B.V., 1105 BP Amsterdam (NL)
(72) Inventor: Van den Ham, René, 1105 BP Amsterdam (NL); Bakker, Bart Jacob, 1105 BP Amsterdam (NL); Arisz, Ryanne Astrid, 1105 BP Amsterdam (NL)
(74) Representative: De Vries & Metman

(57) **Abstract**

A system (1) for determining a functional fibrinogen concentration from a patient's coagulating blood or blood plasma sample (11) is configured to obtain a signal representing physical or biochemical properties of the developing fibrin network during coagulation of the patient's blood or blood plasma sample over time, determine the fibrinogen concentration based on one or more values of one or more features of the signal with the help of a learned function which maps values of the feature(s) to functional fibrinogen concentrations, and output the fibrinogen concentration, e.g. on a display (9). The feature(s) include(s) multiple of features related to 1) a maximum value of the gradient of the signal, 2) the maximum of the second derivative of the signal, 3) a difference between the maximum and the minimum of the signal, and 4) the minimum of the second derivative of the signal, or at least feature 1) or 2).

## Description

### FIELD OF THE INVENTION

The invention relates to systems for determining a functional fibrinogen concentration from a patient's coagulating blood or blood plasma sample and for training a supervised learning model.

The invention further relates to computer-implemented methods of determining a functional fibrinogen concentration from a patient's coagulating blood or blood plasma sample and of training a supervised learning model.

The invention also relates to one or more computer program products enabling a computer system to perform such methods.

### BACKGROUND OF THE INVENTION

Measuring a patient's (functional) fibrinogen concentration is an important part of diagnosing the underlying cause of bleeding in cardiac surgery-, obstetric-, and trauma patients. As these patients need a prompt intervention, the diagnostic test result needs to be available as soon as possible. Tests performed at the central laboratory are often unsuitable as they have a turnaround time of 1 hour or longer. Point-of-care (PoC) tests for fibrinogen are available, but these (viscoelastic) tests only provide a qualitative indication of the fibrinogen concentration (and are expensive).

The current gold (clinical) standard for the quantitative measurement of the fibrinogen concentration is the Clauss assay: a central laboratory test that is based on the clotting time of a test plasma sample when triggered with an excess of thrombin. To derive the fibrinogen concentration in the test plasma, its clotting time is compared to the clotting times of a series of reference samples with increasing, known fibrinogen concentrations. The Clauss method is precise and robust in the sense that it is insensitive to many inhibiting substances (such as anticoagulants and fibrin degradation products). PoC Clauss tests are not available.

Another central laboratory quantitative fibrinogen test is the Prothrombin Time (PT) derived fibrinogen assay. It is based on the maximum change in optical density of a test plasma sample when triggered with a Tissue Factor containing solution. Again, to derive the fibrinogen concentration in the test plasma, its maximum optical density change is compared to the maximum optical density changes of a series of reference samples with increasing, known fibrinogen concentrations. The PT derived Fibrinogen assay is widely used as it comes 'for free' with each PT measurement; it is a second analysis of the transparency over time waveform that is produced by the assay (the first is the determination of the PT; the time at which the plasma sample has reached a predefined transparency). The PT test (together with its standardized form, the International Normalized Ratio (INR)) is the second most performed in vitro diagnostic test worldwide (after glucose) and is also available as a PoC test.

The PT derived Fibrinogen assay in its current form is not considered to be accurate. Especially samples from patients taking anticoagulants are known to give erroneous results, but samples from patients with a prolonged PT in general, or with high fibrinogen levels, as well. Thus, the PT derived Fibrinogen assay is not sufficiently accurate and the Clauss assay cannot be used as PoC test.

### SUMMARY OF THE INVENTION

It is a first object of the invention to provide a system, which is able to determine a functional fibrinogen concentration with sufficient accuracy and can be performed as a PoC test.

It is a second object of the invention to provide a computer-implemented method, which makes it possible to determine a functional fibrinogen concentration with sufficient accuracy and can be performed as a PoC test.

In a first aspect, a system for determining a functional fibrinogen concentration from a patient's coagulating blood or blood plasma sample comprises at least one processor configured to obtain a signal representing physical or biochemical properties of the developing fibrin network during coagulation of the patient's blood or blood plasma sample over time, the signal being determined from the patient's coagulating blood or blood plasma sample, determine the functional fibrinogen concentration based on one or more values of one or more features of the signal with the help of a learned function which maps values of the one or more features to functional fibrinogen concentrations, and output the functional fibrinogen concentration.

The one or more features include at least one feature selected from a first set of features and/or multiple features selected from a larger set of features, the larger set of features comprising the first set of features and a second set of features, the first set of features including a feature related to a maximum value of the gradient of the signal and a feature related to the maximum of the second derivative of the signal, and the second set of features including a feature related to a difference between the maximum and the minimum of the signal and a feature related to the minimum of the second derivative of the signal.

A functional fibrinogen concentration with sufficient accuracy is obtained by not (only) analyzing the change in optical density, but by analyzing one or more other features of the full clotting signal (e.g. as measured by the transparency of the plasma sample over time) that is produced by the assay. This works, because fibrinogen concentration is correlated in a non-trivial way with several features of the signal (which represents physical or biochemical properties of the developing fibrin network during coagulation of the patient's blood or blood plasma sample over time). Like the PT assay, this can be performed as PoC test.

The signal may, for example, represent transparency of the patient's coagulating blood or blood plasma sample over time, dielectric impedance of the patient's coagulating blood or blood plasma sample over time, or viscoelastic properties of the patient's coagulating blood or blood plasma sample over time, or a combination thereof. The system may comprise a measurement component configured to receive the patient's coagulating blood or blood plasma sample and obtain the signal by performing measurements on the patient's coagulating blood or blood plasma sample. The system may be a handheld device, for example.

The at least one processor may be configured to use a trained supervised learning model to determine the functional fibrinogen concentration based on the one or more values of the one or more features of the signal with the help of the learned function, the trained supervised learning model having learned the function based on a set of training examples. Although it may be possible to train a supervised learning model and convert the learned function to a table by inputting all possible values or combinations of values of the one or more features into the supervised learning model, this may be avoided by using the supervised learning model directly in the inference phase. The supervised learning model may comprise a neural network and/or a linear regression model, for example.

Each of the training examples may comprise one or more values of the one or more features determined from a training signal and a measured fibrinogen concentration associated with the training signal. The one or more values of the one or more features of the signal, determined from the patient's coagulating blood or blood plasma sample, may then be input into the trained supervised learning model.

Alternatively, each of the training examples may comprise a training signal and a measured fibrinogen concentration associated with the training signal. The signal, determined from the patient's coagulating blood or blood plasma sample, may then be input into the trained supervised learning model. By determining and inputting the one or more values of the one or more features instead of inputting the (training) signal(s) directly, i.e. by performing pre-processing, the supervised learning model does not need to be trained to determine the one or more values of the one or more features and as a result, training the supervised learning model requires less training examples.

Self-supervised learning methods may be used in conjunction with conventional supervised, e.g. deep, learning methods. In this case, the supervised learning model (or part of the model) may be pre-trained on a task that does not require annotation and the pre-training does in itself not produce a model that solves the task at hand. Instead of a person explicitly specifying/programming the one or more features to be extracted from the signal, self-supervised learning may be used to learn the one or more features to be extracted from the signal.

The at least one processor may be configured to determine the functional fibrinogen concentration by using a first trained supervised learning model to determine a first functional fibrinogen concentration based on the one or more values of the one or more features of the signal, using a second trained supervised learning model to determine a second functional fibrinogen concentration based on the one or more values of the one or more features of the signal, and selecting the first functional fibrinogen concentration or the second functional fibrinogen concentration in dependence on the first functional fibrinogen concentration.

The first trained supervised learning model may comprise a neural network and the second supervised learning model may comprise a linear regression model, for example. For instance, the neural network concentration may be selected by default except when this concentration exceeds a certain threshold, e.g. 6 g/L, in which case the linear regression concentration is selected instead. This may result in more accurate fibrinogen concentration determination.

The at least one processor may be configured to determine a prothrombin-time value related to the prothrombin time of the patient's coagulating blood or blood plasma sample and output a warning along with the determined functional fibrinogen concentration or an error instead of a functional fibrinogen concentration in dependency of the prothrombin-time value. In test results, the correlation of the functional fibrinogen concentrations determined in the manner described above with functional fibrinogen concentrations determined with the Clauss test was found to be weak for certain samples. By determining the PT value and outputting a warning or an error if the PT value is too high to produce very accurate results, e.g. above 60 seconds, users are prevented from using possibly less accurate results (as the results are not output) or are warned not to use the possibly less accurate results or to use the possibly less accurate results with care.

In a second aspect, a computer-implemented method of training a supervised learning model comprises obtaining a set of multiple training examples, each of the training examples comprising a fibrinogen concentration measured from a training coagulating blood or blood plasma sample and at least one of a training signal and one or more values of one or more features of the training signal, the training signal representing physical or biochemical properties of the developing fibrin network during coagulation of the training blood or blood plasma sample over time, the training signal being determined from the training coagulating blood or blood plasma sample, and providing the set of training examples to a supervised learning model to make the supervised learning model learn a function which maps values of the one or more features to functional fibrinogen concentrations.

The one or more features include at least one feature selected from a first set of features and/or multiple features selected from a larger set of features, the larger set of features comprising the first set of features and a second set of features, the first set of features including a feature related to a maximum value of the gradient of the signal and a feature related to the maximum of the second derivative of the signal, and the second set of features including a feature related to a difference between the maximum and the minimum of the signal and a feature related to the minimum of the second derivative of the signal,. The method may be performed by software running on a programmable device. This software may be provided as a computer program product.

The set of training examples may be obtained by obtaining a plurality of training signals and corresponding measured fibrinogen concentrations, analyzing each respective training signal of the training signals to determine the one or more values of the one or more features of the respective training signal from the respective training signal, and including, in the set of training examples, for each respective training signal of the training signals, a training example comprising the one or more values of the one or more features of the respective training signal and the measured fibrinogen concentration associated with the respective training signal.

The supervised learning model may comprise a neural network and/or a linear regression model, for example.

The method may comprise determining, for each training example of the set of training examples, a prothrombin-time value related to the prothrombin time of the training coagulating blood or blood plasma sample, and omitting each training example with a prothrombin-time value exceeding a threshold from the set of training examples. In test results, the correlation of the functional fibrinogen concentrations determined in the manner described above with functional fibrinogen concentrations determined with the Clauss test was found to be weak for certain samples. By omitting training examples with a PT value exceeding a threshold, e.g. 60 seconds, the accuracy of the determined fibrinogen concentration may be increased.

In a third aspect, a system for training a supervised learning model comprises at least one processor configured to obtain a set of multiple training examples, each of the training examples comprising a fibrinogen concentration measured from a training coagulating blood or blood plasma sample and at least one of a training signal and one or more values of one or more features of the training signal, the training signal representing physical or biochemical properties of the developing fibrin network during coagulation of the training blood or blood plasma sample over time, the training signal being determined from the training coagulating blood or blood plasma sample, and providing the set of training examples to a supervised learning model to make the supervised learning model learn a function which maps values of the one or more features to functional fibrinogen concentrations.

The one or more features include at least one feature selected from a first set of features and/or multiple features selected from a larger set of features, the larger set of features comprising the first set of features and a second set of features, the first set of features including a feature related to a maximum value of the gradient of the signal and a feature related to the maximum of the second derivative of the signal, and the second set of features including a feature related to a difference between the maximum and the minimum of the signal and a feature related to the minimum of the second derivative of the signal.

In a fourth aspect, a computer-implemented method of determining a functional fibrinogen concentration from a patient's coagulating blood or blood plasma sample comprises obtaining a signal representing physical or biochemical properties of the developing fibrin network during coagulation of the patient's blood or blood plasma sample over time, the signal being determined from the patient's coagulating blood or blood plasma sample, determining the functional fibrinogen concentration based on one or more values of one or more features of the signal with the help of a learned function which maps values of the one or more features to functional fibrinogen concentrations, and outputting the functional fibrinogen concentration.

The one or more features include at least one feature selected from a first set of features and/or multiple features selected from a larger set of features, the larger set of features comprising the first set of features and a second set of features, the first set of features including a feature related to a maximum value of the gradient of the signal and a feature related to the maximum of the second derivative of the signal, and the second set of features including a feature related to a difference between the maximum and the minimum of the signal and a feature related to the minimum of the second derivative of the signal. The method may be performed by software running on a programmable device. This software may be provided as a computer program product.

Moreover, a computer program for carrying out the methods described herein, as well as a non-transitory computer readable storage-medium storing the computer program are provided. A computer program may, for example, be downloaded by or uploaded to an existing device or be stored upon manufacturing of these systems.

A non-transitory computer-readable storage medium stores at least a first software code portion, the first software code portion, when executed or processed by a computer, being configured to perform executable operations for training a supervised learning model.

These executable operations comprise obtaining a set of multiple training examples, each of the training examples comprising a fibrinogen concentration measured from a training coagulating blood or blood plasma sample and at least one of a training signal and one or more values of one or more features of the training signal, the training signal representing physical or biochemical properties of the developing fibrin network during coagulation of the training blood or blood plasma sample over time, the training signal being determined from the training coagulating blood or blood plasma sample, and providing the set of training examples to a supervised learning model to make the supervised learning model learn a function which maps values of the one or more features to functional fibrinogen concentrations.

The one or more features include at least one feature selected from a first set of features and/or multiple features selected from a larger set of features, the larger set of features comprising the first set of features and a second set of features, the first set of features including a feature related to a maximum value of the gradient of the signal and a feature related to the maximum of the second derivative of the signal, and the second set of features including a feature related to a difference between the maximum and the minimum of the signal and a feature related to the minimum of the second derivative of the signal.

A non-transitory computer-readable storage medium stores at least a second software code portion, the second software code portion, when executed or processed by a computer, being configured to perform executable operations for determining a functional fibrinogen concentration from a patient's coagulating blood or blood plasma sample.

These executable operations comprise obtaining a signal representing physical or biochemical properties of the developing fibrin network during coagulation of the patient's blood or blood plasma sample over time, the signal being determined from the patient's coagulating blood or blood plasma sample, determining the functional fibrinogen concentration based on one or more values of one or more features of the signal with the help of a learned function which maps values of the one or more features to functional fibrinogen concentrations, and outputting the functional fibrinogen concentration.

The one or more features include at least one feature selected from a first set of features and/or multiple features selected from a larger set of features, the larger set of features comprising the first set of features and a second set of features, the first set of features including a feature related to a maximum value of the gradient of the signal and a feature related to the maximum of the second derivative of the signal, and the second set of features including a feature related to a difference between the maximum and the minimum of the signal and a feature related to the minimum of the second derivative of the signal.

As will be appreciated by one skilled in the art, aspects of the present invention may be embodied as a device, a method or a computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit", "module" or "system." Functions described in this disclosure may be implemented as an algorithm executed by a processor/microprocessor of a computer. Furthermore, aspects of the present invention may take the form of a computer program product embodied in one or more computer readable medium(s) having computer readable program code embodied, e.g., stored, thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples of a computer readable storage medium may include, but are not limited to, the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of the present invention, a computer readable storage medium may be any tangible medium that can contain, or store, a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber, cable, RF, etc., or any suitable combination of the foregoing. Computer program code for carrying out operations for aspects of the present invention may be written in any combination of one or more programming languages, including an object oriented programming language such as Java(TM), Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems), and computer program products according to embodiments of the present invention. It will be understood that each block of the flowchart illustrations and/or block diagrams, and combinations of blocks in the flowchart illustrations and/or block diagrams, can be implemented by computer program instructions. These computer program instructions may be provided to a processor, in particular a microprocessor or a central processing unit (CPU), of a general purpose computer, special purpose computer, or other programmable data processing apparatus to produce a machine, such that the instructions, which execute via the processor of the computer, other programmable data processing apparatus, or other devices create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the figures illustrate the architecture, functionality, and operation of possible implementations of devices, methods and computer program products according to various embodiments of the present invention. In this regard, each block in the flowchart or block diagrams may represent a module, segment, or portion of code, which comprises one or more executable instructions for implementing the specified logical function(s).

It should also be noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

### BRIEF DESCRIPTION OF THE DRAWINGS

These and other aspects of the invention are apparent from and will be further elucidated, by way of example, with reference to the drawings, in which:
Fig. 1 is a block diagram of an embodiment of the system;
Fig. 2 shows example of features of a first type of signal;
Fig. 3 is a flow diagram of a first embodiment of the method;
Fig. 4 is a flow diagram of a second embodiment of the method;
Fig. 5 is a flow diagram of a third embodiment of the method;
Fig. 6 is a block diagram of a first embodiment of the supervised learning model;
Fig. 7 is a block diagram of a second embodiment of the supervised learning model;
Fig. 8 is a flow diagram of a third embodiment of the method of training a supervised learning model;
Fig. 9 is a flow diagram of a fourth embodiment of the method;
Fig. 10 is a flow diagram of a fifth embodiment of the method;
Fig. 11 is a flow diagram of a sixth embodiment of the method;
Fig. 12 shows the correlations between measured fibrinogen concentrations and estimations by linear regression;
Fig. 13 shows the correlations between measured fibrinogen concentrations and estimations by a neural network;
Fig. 14 shows the correlations between measured fibrinogen concentrations and estimations by the combination of linear regression and a neural network; and
Fig. 15 is a block diagram of an exemplary data processing system for performing the method of the invention.

Corresponding elements in the drawings are denoted by the same reference numeral.

### DETAILED DESCRIPTION OF THE DRAWINGS

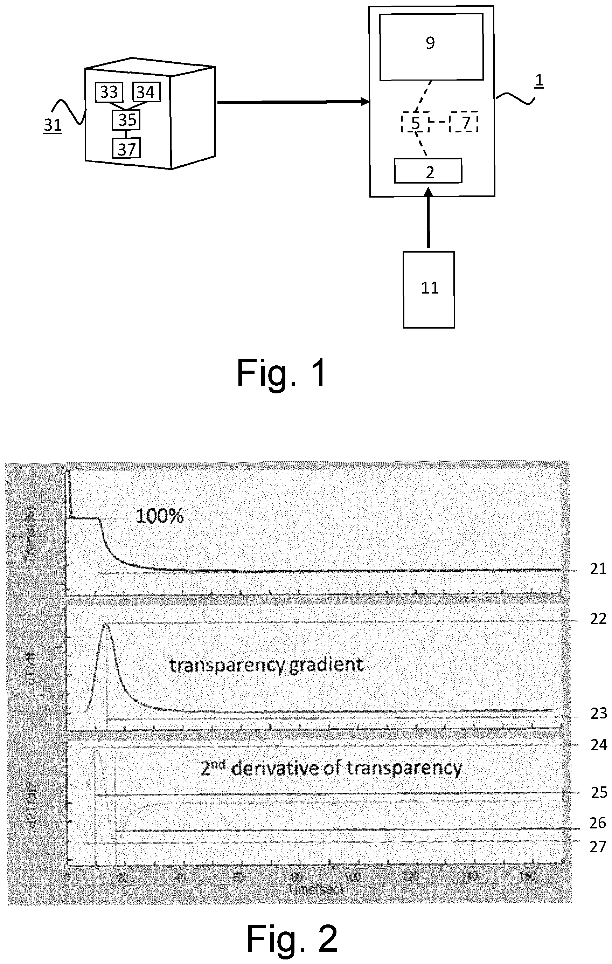

Fig. 1 shows an embodiment of the system for determining a functional fibrinogen concentration from a patient's coagulating blood or blood plasma sample 11. In the embodiment of Fig. 1, the system is a handheld device 1. The handheld device 1 comprises a measurement component 2, a processor 5, a memory 7, and a display 9.

The processor 5 is configured to obtain a signal representing physical or biochemical properties of the developing fibrin network during coagulation of the patient's blood or blood plasma sample over time. In the embodiment of Fig. 1, the handheld device 1 comprises a measurement component 2 configured to receive the patient's coagulating blood or blood plasma sample 11 and obtain the signal by performing measurements on the patient's coagulating blood or blood plasma sample 11. In an alternative embodiment, the measurements are not performed by the device that determines the functional fibrinogen concentration but by another device.

The processor 5 is further configured to determine the functional fibrinogen concentration based on one or more values of one or more features of the signal with the help of a learned function which maps values of the one or more features to functional fibrinogen concentrations. The one or more features include at least one feature selected from a first set of features and/or multiple features selected from a larger set of features. The larger set of features comprises the first set of features and a second set of features.

The first set of features includes a feature b) related to a maximum value of the gradient of the signal and a feature d) related to the maximum of the second derivative of the signal. The second set of features includes a feature a) related to a difference between the maximum and the minimum of the signal and a feature e) related to the minimum of the second derivative of the signal. The one or more features may also include other features, e.g. features c), f), and g). These features are shown in Table 1 below along with examples of these features. The processor 5 is further configured to output the functional fibrinogen concentration, e.g. via the display 9.

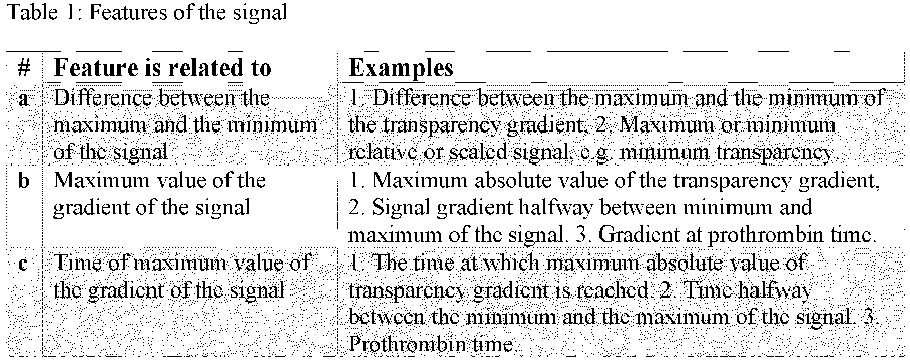

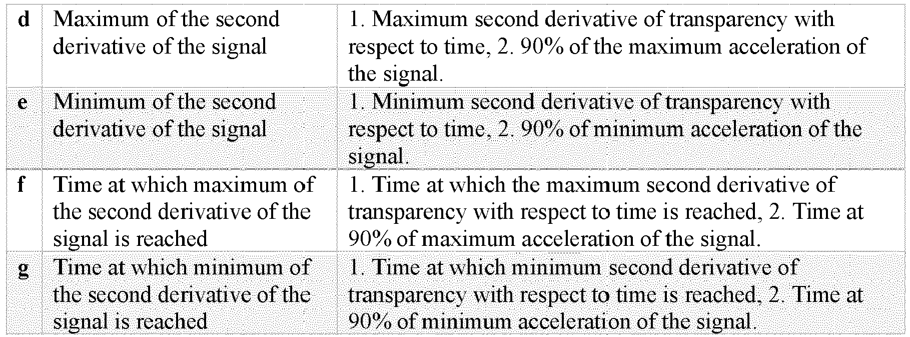

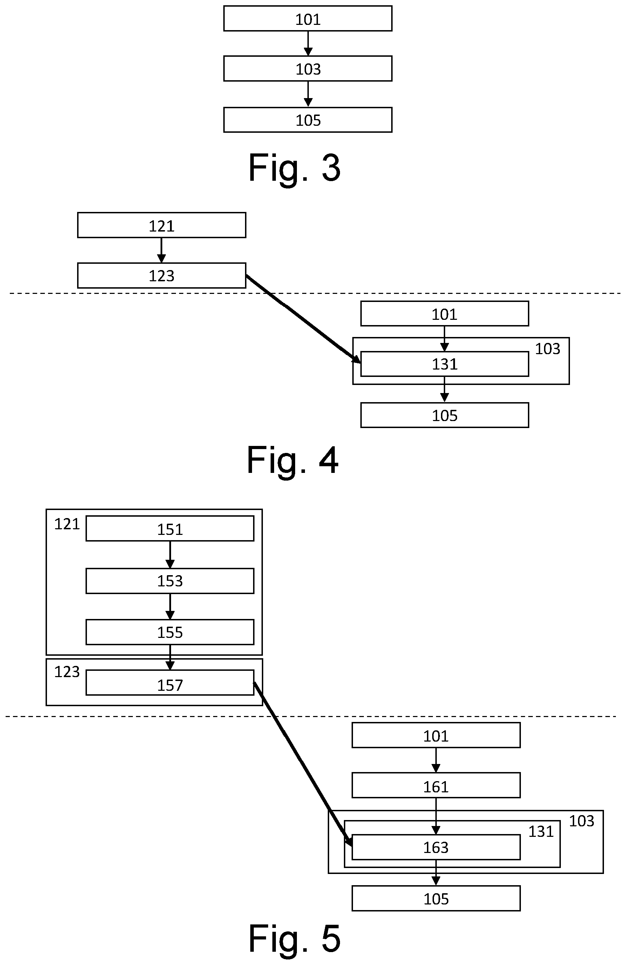

In the example of Fig. 1, the above-mentioned learned function is learned on a (e.g. desktop) computer 31. The computer 31 comprises a processor 35, a receiver 33, a transmitter 34, and a memory 37. The learned function is transferred from the memory 37 of the computer 31 to the memory 7 of the handheld device 1. The processor 35 may, for example, be configured to perform steps 121 and 123 of Fig. 4.

The above-mentioned signal may represent transparency of the patient's coagulating blood or blood plasma sample over time and/or dielectric impedance of the patient's coagulating blood or blood plasma sample over time and/or viscoelastic properties of the patient's coagulating blood or blood plasma sample over time, for example. Fig. 2 shows example of features of a first type of signal, a signal that represent transparency of the patient's coagulating blood or blood plasma sample over time.

In other words, Fig. 2 shows a visualization of transparency signal features, i.e. characteristics of the transparency over time waveform. These characteristics, such as maximum transparency and maximum gradient of the transparency over time, are measured for a specific blood sample and may be used as input to a numerical algorithm that subsequently provides an estimation for the fibrinogen concentration in the sample. The numerical algorithm may be derived from existing data analysis approaches, such as neural network training and linear regression, applied to combinations of measured waveforms and fibrinogen concentrations (e.g., measured via the Clauss method).

Specifically, Fig. 2 shows the transparency signal, the transparency gradient (the 1^{st} derivative of transparency), and the 2^{nd} derivative of transparency. Feature 21 is determined from the transparency signal. Feature 21, minimum transparency, is an example of feature a) of Table 1. Since the maximum transparency is always 100%, the minimum transparency relates to the difference between the maximum and the minimum of the signal.

Features 22 and 23 are determined from the transparency gradient. Feature 22, maximum absolute transparency gradient, is an example of feature b) of Table 1. Feature 23, time of maximum absolute transparency gradient, is an example of feature c) of Table 1.

Features 24-27 are determined from the 2^{nd} derivative of transparency. Feature 24, maximum 2^{nd} derivative of transparency, is an example of feature d) of Table 1. Feature 25, time of maximum of 2^{nd} derivative of transparency, is an example of feature f) of Table 1. Feature 26, time of minimum of 2^{nd} derivative of transparency, is an example of feature g of Table 1. Feature 27, minimum 2^{nd} derivative of transparency, is an example of feature e) of Table 1.

In the embodiment of the computer 1 shown in Fig. 1, the handheld device 1 comprises one processor 5. In an alternative embodiment, the handheld device 1 comprises multiple processors. The processor 5 of the handheld device 1 may be a general-purpose processor, e.g. from e.g. from ARM or Qualcomm, or an application-specific processor. The processor 5 of the handheld device 1 may run a Unix-based operating system for example. The memory 7 may comprise one or more memory units. The memory 7 may comprise solid-state memory, for example. The handheld device 1 may comprise other components typical for a handheld device such as a battery.

In the embodiment of the computer 31 shown in Fig. 1, the computer 31 comprises one processor 35. In an alternative embodiment, the computer 31 comprises multiple processors. The processor 35 of the computer 31 may be a general-purpose processor, e.g. from Intel or AMD, or an application-specific processor. The processor 35 of the computer 31 may run a Windows or Unix-based operating system for example. The memory 37 may comprise one or more memory units. The memory 37 may comprise one or more hard disks and/or solid-state memory, for example. The memory 37 may be used to store an operating system, applications and application data, for example.

The receiver 33 and the transmitter 34 may use one or more wired and/or wireless communication technologies such as Ethernet and/or Wi-Fi (IEEE 802.11), for example. In an alternative embodiment, multiple receivers and/or multiple transmitters are used instead of a single receiver and a single transmitter. In the embodiment shown in Fig. 1, a separate receiver and a separate transmitter are used. In an alternative embodiment, the receiver 33 and the transmitter 34 are combined into a transceiver. The computer 31 may comprise other components typical for a computer such as a power connector.

The invention may be implemented using a computer program running on one or more processors. In the embodiment of Fig. 1, the system is a handheld device. In another embodiment, the system is another type of device, e.g. a desktop computer.

A first embodiment of a computer-implemented method of determining a functional fibrinogen concentration from a patient's coagulating blood or blood plasma sample is shown in Fig. 3. The method may be performed by the handheld device of Fig. 1, for example.

A step 101 comprises obtaining a signal representing physical or biochemical properties of the developing fibrin network during coagulation of the patient's blood or blood plasma sample over time. The signal is or has been determined from the patient's coagulating blood or blood plasma sample.

A step 103 comprises determining the functional fibrinogen concentration based on one or more values of one or more features of the signal with the help of a learned function which maps values of the one or more features to functional fibrinogen concentrations. The one or more features include at least one feature selected from a first set of features and/or multiple features selected from a larger set of features. The larger set of features comprises the first set of features and a second set of features.

The first set of features includes a feature b) related to a maximum value of the gradient of the signal and a feature d) related to the maximum of the second derivative of the signal. The second set of features includes a feature a) related to a difference between the maximum and the minimum of the signal and a feature e) related to the minimum of the second derivative of the signal. The one or more features may also include other features, e.g. features c), f), and g). These features are shown in Table 1.

The function may be learned by applying linear regression to the one or more features and the measured Fibrinogen concentrations or by applying neural network learning on the one or more features and the measured Fibrinogen concentrations, for example. A step 105 comprises outputting the functional fibrinogen concentration, e.g. on a display. Additionally, one or more steps of one or more of the embodiments of Figs. 4-5 and 9-11 may be added to the embodiment of Fig. 3.

A second embodiment of the computer-implemented method of determining a functional fibrinogen concentration from a patient's coagulating blood or blood plasma sample is shown in Fig. 4. The second embodiment of Fig. 4 is an extension of the first embodiment of Fig. 3. Fig. 4 also shows a first embodiment of the computer-implemented method of training a supervised learning model.

A step 121 comprises obtaining a set of multiple training examples. Each of the training examples comprises a fibrinogen concentration measured from a training coagulating blood or blood plasma sample (e.g. by using the Clauss test) and at least one of a training signal and one or more values of one or more features of the training signal. The training signal represents physical or biochemical properties of the developing fibrin network during coagulation of the training blood or blood plasma sample over time.

The training signal is or has been determined from the training coagulating blood or blood plasma sample. The one or more features of the training signal are the same as the one or more features of the signal, which are used in step 103 for determining the functional fibrinogen concentration.

A step 123 comprises providing the set of training examples obtained in step 121 to a supervised learning model to make the supervised learning model learn a function which maps values of the one or more features to functional fibrinogen concentrations. This supervised learning model is used in step 103. The supervised learning model may comprise a neural network and/or a linear regression model, for example. For example, step 123 may comprise applying linear regression or neural network learning to the combination of some or all of the numerical features shown in Fig. 2 and the measured Fibrinogen concentrations. The chosen numerical algorithm and its optimized parameter set may then be stored.

In the embodiments of Fig. 4, step 103 of Fig. 3 is implemented by a step 131. Step 131 comprises using the trained supervised learning model trained in step 123 to determine the functional fibrinogen concentration based on the one or more values of the one or more features of the signal, i.e. the new blood (plasma) sample, with the help of the learned function. Step 131 may comprise applying the chosen numerical algorithm and its optimized parameter set, as stored in step 123.

Steps 121 and 123, which form the training phase, may be performed by the computer 31 of Fig. 1, for example. Steps 101, 131, and 105, which form the inference phase, may be performed by the handheld device of Fig. 1, for example. Additionally, one or more steps of one or more of the embodiments of Figs. 5 and 8-11 may be added to the embodiment of Fig. 4.

In an alternative embodiment, no supervised learning model is used in the inference phase. For example, a lookup table may be used in step 103. The learned function may be encoded in this lookup table. Of course, the more features are used, the larger this lookup table becomes. The values of the lookup table may be learned using a supervised learning model, e.g. a linear regression model.

A third embodiment of a computer-implemented method of determining a functional fibrinogen concentration from a patient's coagulating blood or blood plasma sample is shown in Fig. 5. The third embodiment of Fig. 5 is an extension of the second embodiment of Fig. 4. Fig. 5 also shows a second embodiment of the computer-implemented method of training a supervised learning model. The second embodiment of Fig. 5 is an extension of the first embodiment of Fig. 4.

In the embodiments of Fig. 5, step 121 of Fig. 4 has been implemented by steps 151, 153, and 155, step 123 of Fig. 4 has been implemented by a step 157, step 131 of Fig. 4 has been implemented by a step 163, and a step 161 is performed between steps 101 and 163.

A step 151 comprises obtaining a plurality of training signals and corresponding measured fibrinogen concentrations. A step 153 comprises analyzing each respective training signal of the training signals to determine the one or more values of the one or more features of the respective training signal from the respective training signal. For example, step 151 may comprises collecting measured fibrinogen concentrations and transparency over time waveforms (in blood after activation by e.g., a Tissue Factor containing solution) and step 153 may comprise extracting the waveform features shown in Fig. 2.

A step 155 comprises including, in the set of training examples, for each respective training signal of the training signals, a training example comprising the one or more values of the one or more features of the respective training signal and the measured fibrinogen concentration associated with the respective training signal.

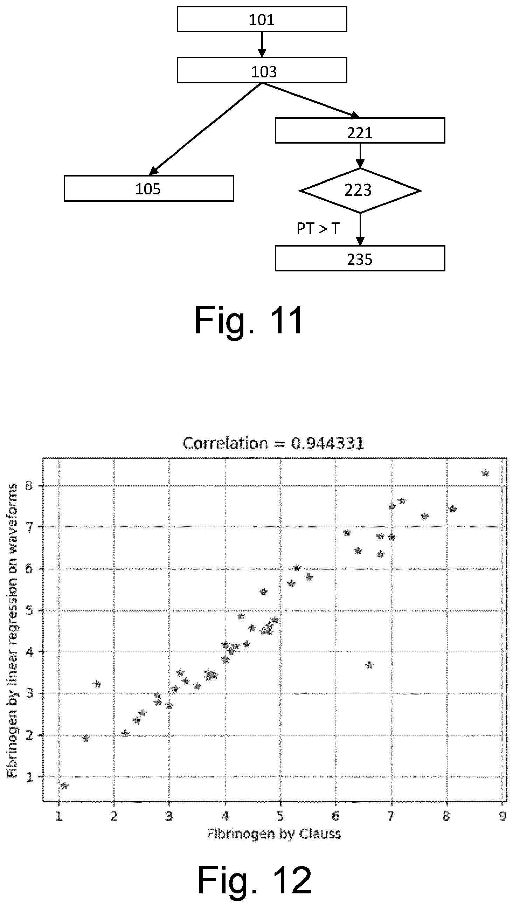

Good results have been obtained (see e.g. Figs. 12-14) when 43 (challenging) samples/training examples were used, of which 24 had a prolonged PT time (>13 seconds), 11 had an anti-F10a test result indicative of anticoagulant use (>0.3 U/ml) or were reported to use anticoagulants and 24 had a high fibrinogen level (> 4 g/l).

Step 157 comprises providing the set of training examples obtained in step 155 to a supervised learning model to make the supervised learning model learn a function which maps values of the one or more features to functional fibrinogen concentrations.

The supervised learning model may comprise a linear regression algorithm applied to features a), b), d), and e) of Table 1, for example. For instance, the following values may be learned/used in the linear regression algorithm:
- Multiplicative coefficients: [-0.06901399, -1.33440313, 8.34123184, - 1.40190392]
- Addition constant (intercept): 6.700150222229663

The supervised learning model may be a 3-layer neural network in which features a), b), d), and e) of Table 1 are used as inputs, for example. This neural network may comprise:
i. An input layer of 4 inputs
ii. A fully connected (or dense) layer with 3 hidden units and tanh as activation function
iii. A fully connected output layer of 1 output with a linear (i.e., no) activation function.

The model inputs may be normalized to zero mean and unit standard deviation in step 153 or 155 and scaled by the same constants in step 161 or 163. The model may be optimized by minimizing the mean squared error between model output and measured fibrinogen concentrations, using Adam optimization with (Keras default) settings learning rate = 0.001, beta_1 = 0.9, beta_2=0.999 and epsilon=1e-7.

The training may, for example, result in the following numerical values (weights and biases) of the trained neural network:
- Weights for the hidden layer:
   [[0.14206399, -1.2544641 , -0.26251107],
   [0.5852421 , 0.03477067, 0.8017831],
   [0.84655154, 0.9527316 , -0.01082217],
   [0.8303179, 0.3308936, 0.64448005]]
- Biases for the hidden layer: [0.35336593, -0.0415823, 0.60492116]
- Weights for the output layer: [1.7333193, 2.3978162, 1.5849648]
- Bias for the output layer: 1.3689603

The determination of the fibrinogen concentration may be further optimized by other choices for the neural network architecture and settings.

Step 101 comprises obtaining a signal representing physical or biochemical properties of the developing fibrin network during coagulation of the patient's blood or blood plasma sample over time. Step 161 comprises determining one or more values of the one or more features of the signal obtained in step 101. For example, the transparency over time waveform may be measured (in blood after activation by e.g., a Tissue Factor containing solution) for the new blood (plasma) sample in step 101 and the same waveform features extracted in step 153 may be extracted in step 161.

Step 163 comprises inputting the one or more values determined in step 161 into the supervised learning model trained in step 157. Thus, step 161 comprises pre-processing the input for the trained supervised learning model. The output of the trained supervised learning model is the functional fibrinogen concentration.

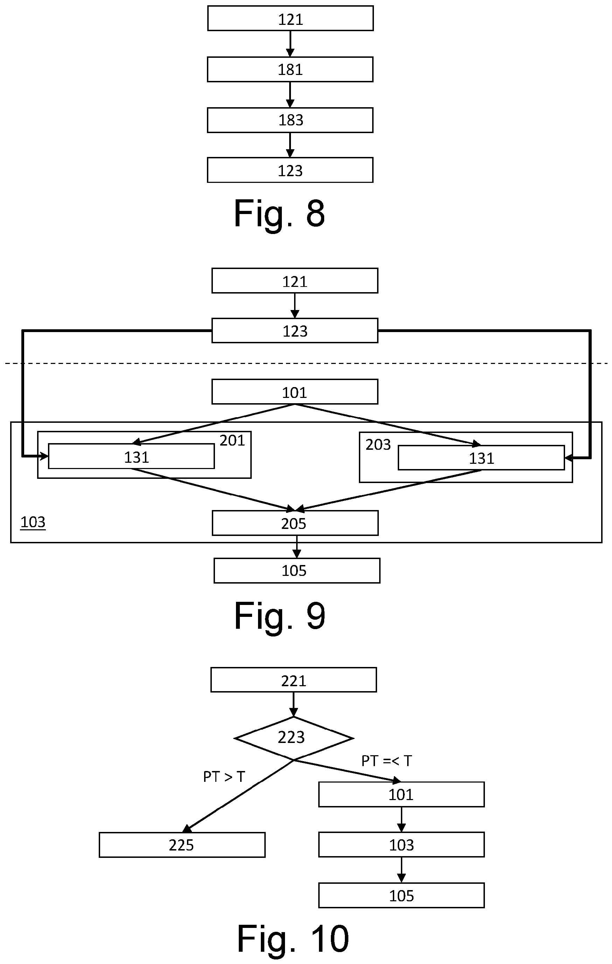

Steps 121 and 123, which form the training phase, may be performed by the computer 31 of Fig. 1, for example. Steps 101, 161, 163, and 105, which form the inference phase, may be performed by the handheld device of Fig. 1, for example. Additionally, one or more steps of one or more of the embodiments of Figs. 8-11 may be added to the embodiment of Fig. 5.

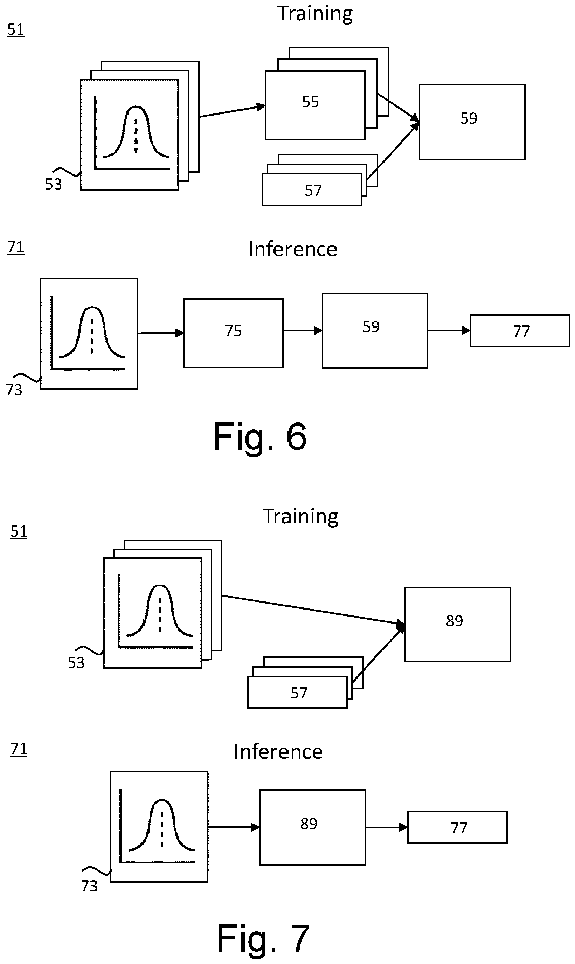

Fig. 6 is a block diagram of the supervised learning model used in the embodiments of Fig. 5. In the training phase 51, training example are fed to a supervised learning model 59. Each training example comprises an input-output pair. This input-output pair comprises training input 55 and corresponding training output 57. In the training phase 51, training signals 53 are analyzed in step 153 of Fig. 5 to determined training input 55. The training input 55 comprises the one or the one or more values of the one or more features of the training signals. The training output 57 comprises the corresponding measured fibrinogen concentrations 57. The training input 55 and the training output 57 are included in the training examples in step 155 of Fig. 5.

In the inference phase 71, input 75 is fed into the supervised learning model 59, which then provides output 77 as a result. The input 75 comprises one or more values of the one or more features of a signal 73, as determined in step 161 of Fig. 5, which are then input in the trained supervised learning model 59 in step 163 of Fig. 5. The output 77 comprises the determined functional fibrogen concentration.

Fig. 7 is a block diagram of a supervised learning model used in an alternative embodiment. In this alternative embodment, the training signals 53 and the signal 73 are not pre-processed but fed/input directly into a supervised learning model 89. The training examples comprise the training signals 53 and the corresponding measured fibrinogen concentrations (training output 57). The output 77 comprises the determined functional fibrogen concentration. This means that the supervised learning model 89 needs to be trained to determine the one or more values of the one or more features of the training signal 53 and of the signal 73. The training of supervised learning model 89 requires more training examples than the training of supervised learning model 59.

A third embodiment of the computer-implemented method of training a supervised learning model is shown in Fig. 8. The method may be performed by the computer 31 of Fig. 1, for example. The third embodiment of Fig. 8 is an extension of the first embodiment of Fig. 4. In the embodiment of Fig. 8, steps 181 and 183 are performed between steps 121 and 123 of Fig. 4.

Step 181 comprises determining, for each training example of the set of training examples, a prothrombin-time value related to the prothrombin time of the training coagulating blood or blood plasma sample. Step 183 comprises omitting each training example with a prothrombin-time value exceeding a threshold, e.g. 60 seconds, from the set of training examples. Additionally, one or more steps of one or more of the embodiments of Figs. 5 and 9 may be added to the embodiment of Fig. 8.

A fourth embodiment of a computer-implemented method of determining a functional fibrinogen concentration from a patient's coagulating blood or blood plasma sample is shown in Fig. 9. The fourth embodiment of Fig. 9 is an extension of the second embodiment of Fig. 4. Fig. 9 also shows a fourth embodiment of the computer-implemented method of training a supervised learning model, which is an extension of the first embodiment of Fig. 4.

In the embodiment of Fig. 9, step 103 of Fig. 4 is implemented by steps 201, 203, and 205. Step 201 comprises using a first trained supervised learning model to determine a first functional fibrinogen concentration based on one or more values of one or more features of the signal. Step 203 comprises using a second trained supervised learning model to determine a second functional fibrinogen concentration based on one or more values of one or more features of the signal.

The values of the same set of features may be used by, e.g. input into, both supervised learning models or the values of a different (possibly overlapping) set of features may be used by, e.g. input into, the different supervised learning models. The first trained supervised learning model may comprise a neural network and the second supervised learning model may comprise a linear regression model, for example. Step 205 comprises selecting the first functional fibrinogen concentration determined in step 201 or the second functional fibrinogen concentration determined in step 203 in dependence on the first functional fibrinogen concentration.

For instance, a neural network prediction may be selected by default, except when the prediction exceeds 6 g/L, in which case a linear regression prediction is selected instead.

Steps 121 and 123, which form the training phase, may be performed by the computer 31 of Fig. 1, for example. Steps 101, 201,203, 205, and 105, which form the inference phase, may be performed by the handheld device of Fig. 1, for example. Additionally, one or more steps of one or more of the embodiments of Figs. 5, 8, and 10-11 may be added to the embodiment of Fig. 9.

A fifth embodiment of a computer-implemented method of determining a functional fibrinogen concentration from a patient's coagulating blood or blood plasma sample is shown in Fig. 10. The fifth embodiment of Fig. 10 is an extension of the first embodiment of Fig. 3. The method may be performed by the handheld device of Fig. 1, for example.

A step 221 comprises determining a prothrombin-time value related to the prothrombin time of the patient's coagulating blood or blood plasma sample. A step 223 comprises checking the prothrombin-time value determined in step 221 and performing step 225 or step 101 in dependency of the prothrombin-time value. For example, step 225 may be performed if the prothrombin-time value exceeds a threshold T, e.g. 60 seconds, and step 101 may be performed the prothrombin-time value does not exceed threshold T.

Step 225 comprises outputting an error instead of a functional fibrinogen concentration. Steps 101-105 have been described in relation to Fig. 3. Step 101 comprises obtaining the signal. Step 103 comprises determining the functional fibrinogen concentration. Step 105 comprises outputting the functional fibrinogen concentration determined in step 103. Additionally, one or more steps of one or more of the embodiments of Figs. 5 and 9 may be added to the embodiment of Fig. 10.

A sixth embodiment of a computer-implemented method of determining a functional fibrinogen concentration from a patient's coagulating blood or blood plasma sample is shown in Fig. 11. The sixth embodiment of Fig. 11 is an extension of the first embodiment of Fig. 3. The method may be performed by the handheld device of Fig. 1, for example.

Step 101 comprises obtaining a signal representing physical or biochemical properties of the developing fibrin network during coagulation of the patient's blood or blood plasma sample over time. Step 103 comprises determining the functional fibrinogen concentration based on one or more values of one or more features of the signal obtained in step 101 with the help of a learned function which maps values of the one or more features to functional fibrinogen concentrations.

Steps 105 and 221 are performed after step 103 has been performed. Step 105 comprises outputting the functional fibrinogen concentration, e.g. on a display. Step 221 comprises determining a prothrombin-time value related to the prothrombin time of the patient's coagulating blood or blood plasma sample. After step 221, step 223 comprises checking the prothrombin-time value determined in step 221 and performing a step 235 in dependency of the prothrombin-time value determined in step 221.

For example, step 235 may be performed if the prothrombin-time value exceeds a threshold T, e.g. 60 seconds. Step 235 comprises output a warning along with the determined functional fibrinogen concentration output in step 105. Additionally, one or more steps of one or more of the embodiments of Figs. 5 and 9 may be added to the embodiment of Fig. 11.

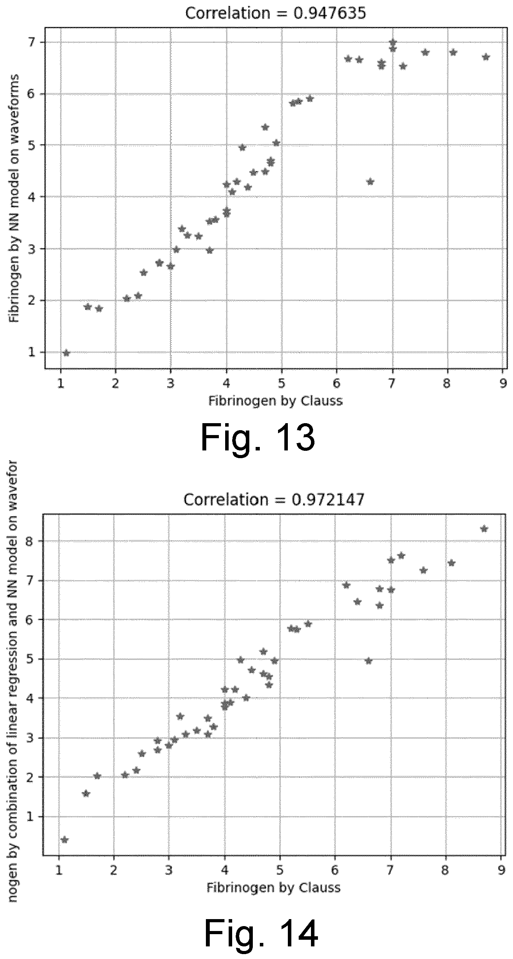

Fig. 12 shows the correlations between measured fibrinogen concentrations and estimations by linear regression. Fig. 13 shows the correlations between measured fibrinogen concentrations and estimations by a neural network. Fig. 14 shows the correlations between measured fibrinogen concentrations and estimations by the combination of linear regression and a neural network.

Figs. 12-14 plot the determined fibrinogen concentrations by linear regression or neural network versus the values as determined/measured with the Clauss method. The linear regression approach achieves a correlation coefficient R of 0.94, while the neural network approach achieves an R of 0.95 on the available data (43 samples). The combination of linear regression and a neural network achieves an R of 0.97. The evaluation is the result of K-fold cross-validation, where K is in this case equal to the total number of samples (43).

Figs. 12-14 shows the results when features a), b), d), and e) of Table 1 are used as inputs. Table 2 shows the results when other features and other combinations of features are used with the afore-mentioned 43 training samples. Table 2 shows the correlation coefficients per combination of features for direct correlation between the (single) feature and Fibrinogen level, and between the output of linear regression / the neural network and Fibrinogen.

Overall, the results indicate feature a (feature related to difference between the maximum and the minimum of the signal) to be the strongest feature, feature e (feature related to minimum of the second derivative of the signal) the least strong of the features a, b, d, and e, and features b and d (feature related to maximum value of the gradient of the signal and feature related to maximum of the second derivative of the signal) about equally strong.

**Table 2: Correlation coefficient between estimated and measured Fibrinogen level**

| Features | Direct correlation | Correlation under linear regression | Correlation of neural network output | Correlation of combined model |
|---|---|---|---|---|
| Feature a | 0.92 | 0.90 | 0.63 | 0.63 |
| Feature b | 0.95 | 0.94 | 0.91 | 0.93 |
| Feature c | 0.03 | | | |
| Feature d | 0.94 | 0.93 | 0.85 | 0.86 |
| Feature e | 0.86 | 0.84 | 0.77 | 0.81 |
| Feature f | 0.03 | | | |
| Feature g | 0.03 | | | |
| a&b | | 0.96 | 0.93 | 0.96 |
| a&d | | 0.96 | 0.96 | 0.97 |
| a&e | | 0.97 | 0.93 | 0.95 |
| b&d | | 0.94 | 0.92 | 0.92 |
| b&e | | 0.96 | 0.87 | 0.90 |
| d&e | | 0.96 | 0.87 | 0.91 |
| a&b&d | | 0.96 | 0.95 | 0.97 |
| a&b&e | | 0.95 | 0.94 | 0.96 |
| a&d&e | | 0.95 | 0.93 | 0.95 |
| b&d&e | | 0.95 | 0.89 | 0.92 |
| a&b&d&e | | 0.94 | 0.95 | 0.97 |

Table 3 shows the improved results when a sample with a PT value exceeding a threshold (the outlier) is omitted from the set of training examples, as described in relation to Fig. 8.

**Table 3: Results with outlier removed**

| Features | Direct correlation | Correlation under linear regression | Correlation of neural network output | Correlation of combined model |
|---|---|---|---|---|
| Feature a | 0.92 | 0.91 | 0.49 | 0.49 |
| Feature b | 0.98 | 0.98 | 0.92 | 0.94 |
| Feature c | 0.20 | | | |
| Feature d | 0.98 | 0.98 | 0.92 | 0.93 |
| Feature e | 0.92 | 0.92 | 0.86 | 0.86 |
| Feature f | 0.19 | | | |
| Feature g | 0.19 | | | |
| a & b | | 0.98 | 0.97 | 0.98 |
| a & d | | 0.98 | 0.96 | 0.98 |
| a & e | | 0.98 | 0.96 | 0.97 |
| b & d | | 0.98 | 0.94 | 0.97 |
| b & e | | 0.98 | 0.93 | 0.96 |
| d & e | | 0.99 | 0.94 | 0.97 |
| a & b & d | | 0.98 | 0.97 | 0.98 |
| a & b & e | | 0.98 | 0.96 | 0.98 |
| a & d & e | | 0.99 | 0.96 | 0.98 |
| b & d & e | | 0.99 | 0.93 | 0.97 |
| a & b & d & e | | 0.99 | 0.96 | 0.98 |

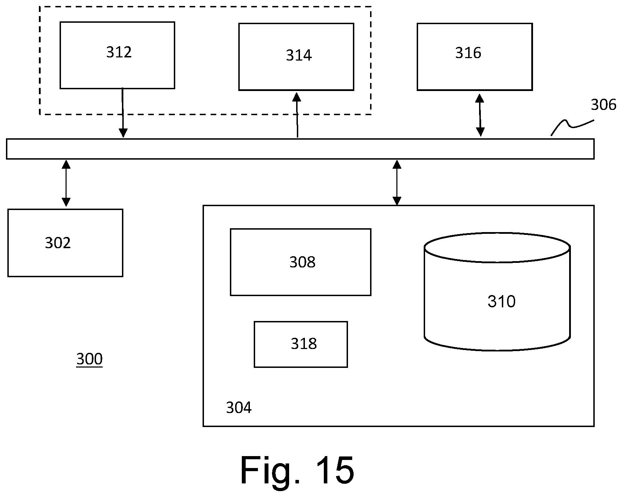

Fig. 15 depicts a block diagram illustrating an exemplary data processing system that may perform the method as described with reference to Figs. 3-5 and 8-11.

As shown in Fig. 15, the data processing system 300 may include at least one processor 302 coupled to memory elements 304 through a system bus 306. As such, the data processing system may store program code within memory elements 304. Further, the processor 302 may execute the program code accessed from the memory elements 304 via a system bus 306. In one aspect, the data processing system may be implemented as a computer that is suitable for storing and/or executing program code. It should be appreciated, however, that the data processing system 300 may be implemented in the form of any system including a processor and a memory that is capable of performing the functions described within this specification.

The memory elements 304 may include one or more physical memory devices such as, for example, local memory 308 and one or more bulk storage devices 310. The local memory may refer to random access memory or other non-persistent memory device(s) generally used during actual execution of the program code. A bulk storage device may be implemented as a hard drive or other persistent data storage device. The processing system 300 may also include one or more cache memories (not shown) that provide temporary storage of at least some program code in order to reduce the number of times program code must be retrieved from the bulk storage device 310 during execution.

Input/output (I/O) devices depicted as an input device 312 and an output device 314 optionally can be coupled to the data processing system. Examples of input devices may include, but are not limited to, a keyboard, a pointing device such as a mouse, or the like. Examples of output devices may include, but are not limited to, a monitor or a display, speakers, or the like. Input and/or output devices may be coupled to the data processing system either directly or through intervening I/O controllers.

In an embodiment, the input and the output devices may be implemented as a combined input/output device (illustrated in Fig. 15 with a dashed line surrounding the input device 312 and the output device 314). An example of such a combined device is a touch sensitive display, also sometimes referred to as a "touch screen display" or simply "touch screen". In such an embodiment, input to the device may be provided by a movement of a physical object, such as e.g. a stylus or a finger of a user, on or near the touch screen display.

A network adapter 316 may also be coupled to the data processing system to enable it to become coupled to other systems, computer systems, remote network devices, and/or remote storage devices through intervening private or public networks. The network adapter may comprise a data receiver for receiving data that is transmitted by said systems, devices and/or networks to the data processing system 300, and a data transmitter for transmitting data from the data processing system 300 to said systems, devices and/or networks. Modems, cable modems, and Ethernet cards are examples of different types of network adapter that may be used with the data processing system 300.

As pictured in Fig. 15, the memory elements 304 may store an application 318. In various embodiments, the application 318 may be stored in the local memory 308, he one or more bulk storage devices 310, or separate from the local memory and the bulk storage devices. It should be appreciated that the data processing system 300 may further execute an operating system (not shown in Fig. 15) that can facilitate execution of the application 318. The application 318, being implemented in the form of executable program code, can be executed by the data processing system 300, e.g., by the processor 302. Responsive to executing the application, the data processing system 300 may be configured to perform one or more operations or method steps described herein.

Various embodiments of the invention may be implemented as a program product for use with a computer system, where the program(s) of the program product define functions of the embodiments (including the methods described herein). In one embodiment, the program(s) can be contained on a variety of non-transitory computer-readable storage media, where, as used herein, the expression "non-transitory computer readable storage media" comprises all computer-readable media, with the sole exception being a transitory, propagating signal. In another embodiment, the program(s) can be contained on a variety of transitory computer-readable storage media. Illustrative computer-readable storage media include, but are not limited to: (i) non-writable storage media (e.g., read-only memory devices within a computer such as CD-ROM disks readable by a CD-ROM drive, ROM chips or any type of solid-state non-volatile semiconductor memory) on which information is permanently stored; and (ii) writable storage media (e.g., flash memory, floppy disks within a diskette drive or hard-disk drive or any type of solid-state random-access semiconductor memory) on which alterable information is stored. The computer program may be run on the processor 302 described herein.

The terminology used herein is for the purpose of describing particular embodiments only and is not intended to be limiting of the invention. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The description of embodiments of the present invention has been presented for purposes of illustration, but is not intended to be exhaustive or limited to the implementations in the form disclosed. Many modifications and variations will be apparent to those of ordinary skill in the art without departing from the scope of the present invention. The embodiments were chosen and described in order to best explain the principles and some practical applications of the present invention, and to enable others of ordinary skill in the art to understand the present invention for various embodiments with various modifications as are suited to the particular use contemplated.

## Claims

1. A system (1) for determining a functional fibrinogen concentration from a patient's coagulating blood or blood plasma sample (11), the system (1) comprising at least one processor (5) configured to:
- obtain a signal representing physical or biochemical properties of the developing fibrin network during coagulation of the patient's blood or blood plasma sample over time, the signal being determined from the patient's coagulating blood or blood plasma sample (11);
- determine the functional fibrinogen concentration based on one or more values of one or more features of the signal with the help of a learned function which maps values of the one or more features to functional fibrinogen concentrations, the one or more features including at least one feature selected from a first set of features and/or multiple features selected from a larger set of features, the larger set of features comprising the first set of features and a second set of features,
the first set of features including a feature (22) related to a maximum value of the gradient of the signal and a feature (24) related to the maximum of the second derivative of the signal, and
the second set of features including a feature (21) related to a difference between the maximum and the minimum of the signal and a feature (27) related to the minimum of the second derivative of the signal; and
- output the functional fibrinogen concentration.

2. A system (1) as claimed in claim 1, wherein the signal represents transparency of the patient's coagulating blood or blood plasma sample over time, dielectric impedance of the patient's coagulating blood or blood plasma sample over time, and/or viscoelastic properties of the patient's coagulating blood or blood plasma sample over time.

3. A system (1) as claimed in claim 2, wherein the at least one processor (5) is configured to use a trained supervised learning model to determine the functional fibrinogen concentration based on the one or more values of the one or more features of the signal with the help of the learned function, the trained supervised learning model having learned the function based on a set of training examples.

4. A system (1) as claimed in claim 3, wherein each of the training examples comprises one or more values of the one or more features determined from a training signal and a measured fibrinogen concentration associated with the training signal and the one or more values of the one or more features of the signal, determined from the patient's coagulating blood or blood plasma sample (11), are input into the trained supervised learning model.

5. A system (1) as claimed in claim 3, wherein each of the training examples comprises a training signal and a measured fibrinogen concentration associated with the training signal and wherein the signal, determined from the patient's coagulating blood or blood plasma sample (11), is input into the trained supervised learning model.

6. A system (1) as claimed in any one of claims 3 to 5, wherein the supervised learning model comprises a neural network and/or a linear regression model.

7. A system (1) as claimed in any one of claims 3 to 6, wherein the at least one processor (5) is configured to determine the functional fibrinogen concentration by:
- using a first trained supervised learning model to determine a first functional fibrinogen concentration based on the one or more values of the one or more features of the signal,
- using a second trained supervised learning model to determine a second functional fibrinogen concentration based on the one or more values of the one or more features of the signal, and
- selecting the first functional fibrinogen concentration or the second functional fibrinogen concentration in dependence on the first functional fibrinogen concentration.

8. A system (1) as claimed in claim 7, wherein the first trained supervised learning model comprises a neural network and the second supervised learning model comprises a linear regression model.

9. A system (1) as claimed in any one of the preceding claims, wherein the at least one processor (5) is configured to determine a prothrombin-time value related to the prothrombin time of the patient's coagulating blood or blood plasma sample (11) and to output a warning along with the determined functional fibrinogen concentration or an error instead of a functional fibrinogen concentration in dependency of the prothrombin-time value.

10. A system (1) as claimed in any one of the preceding claims, wherein the system (1) comprises a measurement component (2) configured to receive the patient's coagulating blood or blood plasma sample (11) and obtain the signal by performing measurements on the patient's coagulating blood or blood plasma sample (11).

11. A computer-implemented method of training a supervised learning model, the method comprising:
- obtaining (121) a set of multiple training examples, each of the training examples comprising a fibrinogen concentration measured from a training coagulating blood or blood plasma sample and at least one of a training signal and one or more values of one or more features of the training signal,
the training signal representing physical or biochemical properties of the developing fibrin network during coagulation of the training blood or blood plasma sample over time, the training signal being determined from the training coagulating blood or blood plasma sample,
the one or more features including at least one feature selected from a first set of features and/or multiple features selected from a larger set of features, the larger set of features comprising the first set of features and a second set of features,
the first set of features including a feature related to a maximum value of the gradient of the signal and a feature related to the maximum of the second derivative of the signal, and
the second set of features including a feature related to a difference between the maximum and the minimum of the signal and a feature related to the minimum of the second derivative of the signal; and
- providing (123) the set of training examples to a supervised learning model to make the supervised learning model learn a function which maps values of the one or more features to functional fibrinogen concentrations.

12. A method as claimed in claim 11, wherein the set of training examples is obtained by:
- obtaining (151) a plurality of training signals and corresponding measured fibrinogen concentrations;
- analyzing (153) each respective training signal of the training signals to determine the one or more values of the one or more features of the respective training signal from the respective training signal; and
- including (155), in the set of training examples, for each respective training signal of the training signals, a training example comprising the one or more values of the one or more features of the respective training signal and the measured fibrinogen concentration associated with the respective training signal.

13. A method as claimed in claim 11 or 12, wherein the supervised learning model comprises a neural network and/or a linear regression model.

14. A method as claimed in any one of claims 11 to 13, wherein the method comprises:
- determining (181), for each training example of the set of training examples, a prothrombin-time value related to the prothrombin time of the training coagulating blood or blood plasma sample; and
- omitting (183) each training example with a prothrombin-time value exceeding a threshold from the set of training examples.

15. A computer program or suite of computer programs comprising at least one software code portion or a computer program product storing at least one software code portion, the software code portion, when run on a computer system, being configured for performing the method of any one of claims 11 to 14 or for behaving like the system of any one of claims 1 to 10.
